# EUROPEAN PATENT APPLICATION

(11) **EP 1 826 263 A1**
(43) Date of publication of application: **29.08.2007**
(21) Application number: 07290216.6
(22) Date of filing: 20.02.2007
(51) Int. Cl.: C12M 1/12

(54) **Microbiological test device, test and incubation systems including it, and method using it**

(30) Priority: 24.02.2006 FR 0650647
(71) Applicant: MILLIPORE CORPORATION, Billerica Massachusetts 01821 (US)
(72) Inventor: Kieffer, Vincent, 67700 Otterswiller (FR); Dorninger, Thibault, 67280 Urmatt (FR); Waiche, Gaël, 67120 Molsheim (FR); Olivier, Stéphane, 67560 Rosheimh (FR)
(74) Representative: Santarelli

(57) **Abstract**

The device includes a first body including an inlet orifice, an outlet orifice (21), a filter membrane (3), a support member (4) for the membrane (3) that is removable from said first body (2) and a second body (6) removably fixed to said first body (2); the outlet orifice (21) is formed at the periphery of said first body (2) and a second body (6) is primarily formed of a detachable wall, mounted by adhesion to said first body (2).

The test assembly includes a device of the above kind and a clamp adapted to grip said device n the manner of a vice.

The incubation assembly includes a device of the above kind and a gel growth medium cassette.

The method includes steps of mounting a device of the above kind in a clamp, filtering a volume of liquid in the device, nesting said device to a gel growth medium cassette and waiting for incubation to take place.

## Description

The present invention relates to the microbiological testing of a sample of liquid flowing under pressure.

Devices for microbiological testing of liquid samples under pressure including a microporous membrane through which the liquid under pressure is filtered are already known in the art, in particular from French patent 2 802 942. These devices include, on the one hand, an inlet body having an inlet orifice and a cup adapted to receive the liquid entering via the inlet orifice before it is filtered through the membrane and, on the other hand, a drainage body separate from the inlet body, adapted to support the membrane on the side opposite the cup and having a filtered liquid outlet orifice.

The inlet body and the drainage body lock together with the filter membrane between them in a configuration in which they clamp the membrane annularly via a seal.

This locking is obtained by complementary clipping means on the inlet body and the drainage body designed to resist the pressure of the liquid in the chamber formed by the cup and the membrane during filtration.

Moreover, as these pressures can be as high as several bar, the drainage and inlet bodies must have mechanical stiffness characteristics (for example in terms of thickness) able to withstand these pressure stresses.

The invention aims to provide a microbiological testing device that is more economic, simpler and more convenient to fabricate and use than the prior art devices.

To this end a first aspect of the invention consists in a device for microbiological testing of a sample of liquid flowing under pressure in an axial filtration direction, the device including:
- a first body having an inlet orifice and a receiving volume connected to said inlet orifice;
- an outlet orifice and a drainage volume communicating with said outlet orifice;
- a filter membrane disposed transversely to said axial direction between the receiving volume and the drainage volume;
- a support member removable from said first body and adapted to restrict deformation of the membrane when the liquid flows from the receiving volume) to the drainage volume without blocking that flow; and
- a second body removably fixed to said first body to delimit the drainage volume conjointly with the membrane;
characterized in that:
- the outlet orifice is at the periphery of said first body; and
- said second body is primarily formed of a detachable wall mounted by adhesion to said first body.

Here the expression "wall mounted by adhesion" means any member detachably retained by friction forces (for example by force-fitting) or bonding forces (for example by adhesive bonding). Detachment is therefore effected without breakage outside the adhesion region.

The detachable wall guarantees the integrity of the device, protecting the membrane from external contamination. Simple retention by adhesion is sufficient to guarantee this integrity and enables the use of a simplified structure for the second body mounted by adhesion to the first body and having no clipping means that are complex to mould and therefore costly to fabricate.

It should be noted that virtually no forces are applied to the interface between the first body and the detachable wall during storage prior to use or during preparation for use, with the result that the connection to this interface does not need to be very strong. Moreover, the risk of accidental tearing of the second body from the first body is low, the regions for the operator to grip on the second body being restricted (being formed primarily of a simple wall).

When the device is to be used, the structural simplicity of its detachable wall enables the use of a clamp, the detachable wall then being also adapted to withstand the high pressure of the liquid that flows through the device by virtue of the reinforcement provided by the clamp.

Clearly the use of a clamp of this kind means that the stiffness and thus the weight and overall size of the first body can also be reduced.

Diverse features of the invention may be implemented for reasons of simplicity, convenience and economy of use, and where appropriate combined with each other.

Thus according to one advantageous feature of the invention, at least the major portion of said drainage volume is formed in said first body. The advantageous grouping together within the first body of the receiving and drainage volumes optimizes the space delimited by this body, making the device more compact. This compactness in particular means that the mass of material to be used and therefore the fabrication cost of a device of this kind can be reduced. Incorporating different volumes in the same component does not make it too complex, the component remaining reasonably simple and convenient to mould in one piece.

According to other advantageous features of the invention of the invention, said detachable wall holds said supporting member in position inside said first body and said detachable wall is advantageously sealed to said support member. As a result, when the detachable wall is separated from the first body, the support member is then no longer held in position by the detachable wall and can therefore be entrained with it, so that the filter membrane is rendered accessible with no risk of contamination by contact during this operation.

According to other advantageous features of the invention:
- said wall of the second body is flexible, which facilitates detaching it; it is advantageously a peelable film, in practice fixed by a layer of adhesive; alternatively, said detachable wall is part of a cover force-fitted into or onto said first body;
- said first body has on the opposite side of the wall of the second body to said membrane a second detachable wall mounted by adhesion to said first body and closing said receiving volume, which can facilitate the construction of the body;
- said receiving volume forms a closed space in the presence of said second detachable wall, which protects the receiving volume from external contamination during filtering and during incubation of the microorganisms; it is only after incubation that this second wall may be detached, if necessary, to view and count microorganisms that have been grown on the membrane;
- said second wall is flexible, which may proves useful after the incubation phase; it is a peelable film, for example, but is advantageously part of a cover force-fitted into or onto said first body; said second wall advantageously includes a flexible hinge which advantageously includes at least one portion of thinner material; the cover preferably has a first stable position in which said receiving volume occupies a predetermined space and a second stable position in which said receiving volume occupies a smaller space than said predetermined space; this cover being deformable, depressing it generates a slight pressure rise in the receiving volume that deforms the membrane, if the supporting member has been removed, imparting a convex shape to it to facilitate its application to a gel growth medium, for example;
- said walls mounted by adhesion to said first body are substantially parallel to each other, which simplifies the geometry of the clamp, although more complex shapes are compatible with gripping by a clamp.

According to another advantageous feature of the invention of the invention, said first body has on the opposite side of the wall of the second body to said membrane a second wall parallel to said detachable wall, without which this wall is not necessarily detachable.

According to other advantageous features of the invention of the invention:
- the periphery of said membrane is held against an annular rim of said first body between said receiving volume and a peripheral transit volume through which said drainage volume communicates with the outlet orifice, the transit volume collecting the liquid coming from the drainage volume in a reduced volume inside the first body; this membrane is advantageously sealed to said annular rim, which provides a sealed connection without having to provide rigorous clamping; said rim is part of a globally frustoconical wall that converges axially toward the membrane; the transit volume is advantageously situated between said rim, a lateral wall around said rim and a transverse wall connecting said rim to said lateral wall on the opposite axial side to the detachable wall, so that the transit volume is accommodated optimally in the first body; the drainage volume extends transversely beyond the annular rim to join said peripheral transit volume, which makes the device more compact; said support member is advantageously a porous member that extends laterally at least to a position facing said rim and which extends axially over more than half of the distance between said membrane and said detachable wall, which helps to drain the liquid to the transit volume in a small space;
- said first body has an annular shape that is globally symmetrical in the axial direction, so that the membrane is optimally surrounded;
- said support member is porous;
- said device includes a holding member disposed between said support member and said detachable wall, which holds the support member against the first body;
- said device includes a first calibrated valve disposed between said inlet orifice and said receiving volume and a second calibrated valve disposed between said drainage volume and said outlet orifice, said valves being adapted to open at said flow pressure of the liquid in the flow direction from said inlet orifice to said outlet orifice, which guarantees the integrity of the membrane and closes the receiving volume which is thus protected by external contamination.

A second aspect of the invention consists in an assembly for microbiological testing of a sample of liquid flowing under pressure including the above device and a clamp having clamping means adapted to hold said device axially in the manner of a vice by said first body and said detachable wall.

Thus the pressure stresses exerted on the device are absorbed mechanically by the clamp. Unlike the device itself, which is disposable, this clamp can be used again for each new device, which helps to reduce the overall cost in terms of quantities of materials.

Using a clamp also enables the device to resist high flow pressures.

Diverse features of the invention may be implemented for reasons of simplicity, convenience and economy of use, and where appropriate combined with each other.

According to one advantageous feature of the invention, said clamp exerts a clamping pressure on said detachable wall, which maintains the sealing and the integrity of the device despite the high flow pressures; to this end, for example, said clamp has a fixed plate and a plate mobile between a rest position in which it is moved away from the device and a clamping position in which it bears on said device and said clamping means include a knob and a clamping mechanism, said knob being adapted to be operated to operate said clamping mechanism to move the mobile plate from its rest position to its clamping position and vice versa; said clamping mechanism preferably is adapted to limit the clamping torque when said knob is operated so that said clamp exerts a predetermined clamping pressure on said device, so the device can be clamped with as pressure selected so as not to crush the device to no good purpose but clamping it sufficiently to guarantee sealing of the device; the plate that engages said detachable wall advantageously has a central cavity, which, if this cavity is empty, allows the detachable wall to deform into the cavity to increase the drainage volume and, if said cavity is filled with an elastic and flexible material, for example a silicone, strengthens the seal in the region in which the device bears on the silicone.

A third aspect of the invention consists in a microbiological incubation assembly including a device as defined hereinabove and a gel growth medium cassette including a body surrounding said gel growth medium and a support on which said gel growth medium rests, having on the side opposite said support a convex surface, the device being adapted to be nested with said cassette after removal of said detachable wall and of said support member so that, in the position of nesting of said device in said cassette, the surface of the membrane of said device facing said cassette rests on said gel growth medium.

This cassette is therefore designed to allow the membrane to come into contact with the gel growth medium over the whole of its surface without having to separate the membrane from the test device, the receiving volume thus being protected from external contamination during this operation.

Diverse features of the invention may be implemented for reasons of simplicity, convenience and economy of use, and where appropriate combined with each other.

According to advantageous features of the invention:
- said support is a mesh onto which said gel growth medium is poured;
- said device and said cassette include complementary clipping means, which enables stable nesting of the device and the cassette; these complementary clipping means include, for example, at least one boss forming part of said device and one groove forming part of said cassette.

A fourth aspect of the invention consists in a method for microbiological testing of a sample of liquid flowing under pressure, including steps of:
- procuring a test device as defined hereinabove;
- mounting said device in a clamp to constitute the above test assembly;
- connecting the inlet orifice of said device to a filler passage and the outlet orifice to a drainage passage for said liquid;
- filtering a volume of liquid through said device;
- removing said device from said clamp;
- purging the liquid from said device;
- removing said detachable wall and said support member from said device;
- nesting said device on a gel growth medium cassette to constitute an incubation assembly according to any one of claims 36 to 39; and
- waiting for incubation to take place.

This method therefore provides an efficient, convenient and economical way to carry out a microbiological test, from filtration to incubation, in the same device.

Diverse features of the invention may be implemented for reasons of simplicity, convenience and economy of use, and where appropriate combined with each other.

According to an advantageous feature of the invention, the step of procuring a test assembly includes the step of selecting as the test device a device having a first body including at least one flexible wall on the opposite side of the detachable wall of the second body to said membrane and in that said method includes, prior to the step of nesting the device to the gel growth medium cassette, the steps of:
- pressing on said first body to flex said wall toward the interior of said device so that said membrane is domed toward the exterior of said device; and
- pressing the membrane onto the gel growth medium, bringing it into contact with the gel growth medium firstly at its center and then progressively over the whole of its surface out to its periphery.

The progressive application of the membrane to the gel growth medium from its centre to its periphery progressively expels air situated between the membrane and the gel growth medium to minimize the risk of residual air pockets remaining between the membrane and the gel growth medium.

According to another advantageous feature of the invention, the step of purging said liquid includes the step of connecting the outlet orifice of said device to a vacuum supply and the step of aspirating said liquid through said outlet orifice.

The explanation of the invention continues with the detailed description of one embodiment of the invention given hereinafter by way of illustrative and nonlimiting example and with reference to the appended drawings. In the drawings:
- figure 1 is a perspective view of a microbiological test device of the invention;
- figure 2 is a similar exploded view of the device;
- figure 3 is a top plan view of the device;
- figure 4 is an elevation view of the device in section taken along the line IV-IV in figure 3;
- figure 5 is a view similar to figure 4 but showing a body of the device separately;
- figure 6 is a view to a larger scale of the detail VI in figure 4;
- figure 7 is a perspective view showing a clamp into which the device has been introduced to be clamped therein in the manner of a vice;
- figures 8 and 9 are two elevation views in section of the device engaged in the clamp, respectively showing the clamp in a position in which the mobile clamping plate of the clamp is moved away from the device and a position in which that plate is clamped against the device;
- figure 10 is a perspective view of a gel growth medium cassette designed to be used in conjunction with the microbiological test device when the liquid has been filtered;
- figure 11 is a similar exploded view of the figure 10 cassette;
- figure 12 is an elevation view of the cassette in section;
- figures 13 to 15 are three elevation views in section respectively showing an approach phase, a bringing into contact phase and a phase of clipping the device to the cassette; and
- figure 16 is a perspective view of the assembly formed by the cassette and the device when clipped together.

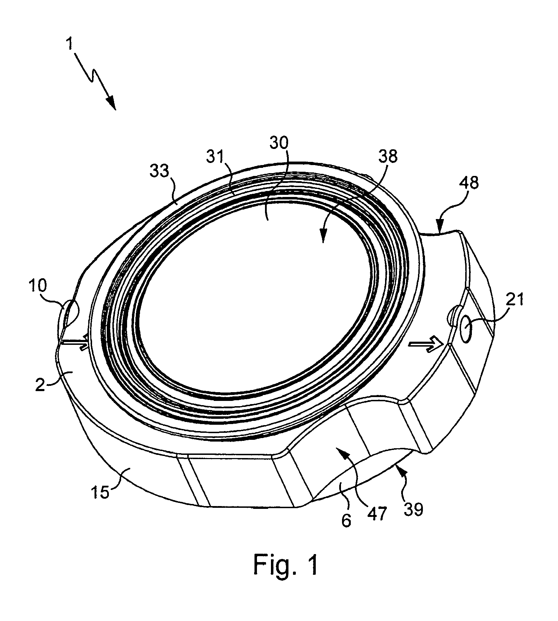

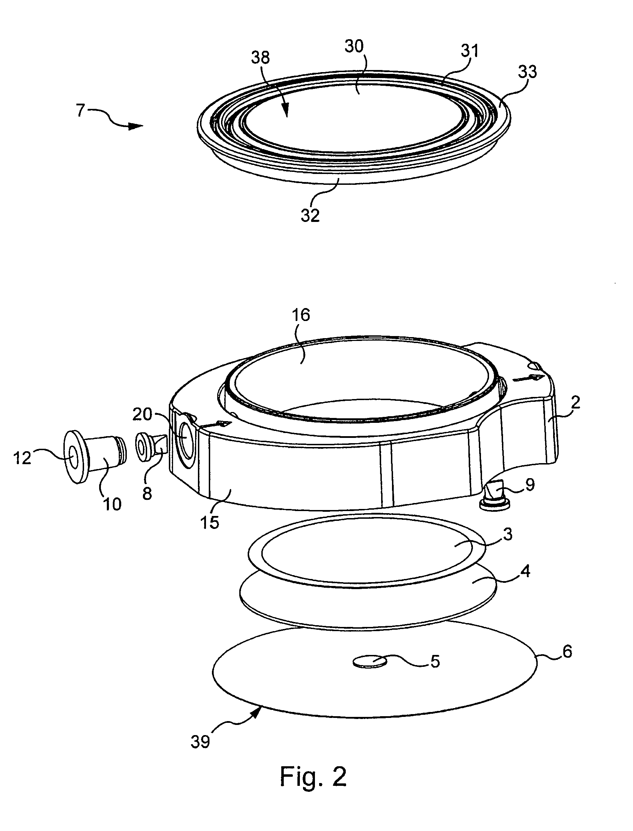

The microbiological test device 1 represented in figures 1 to 3 comprises a body 2, a membrane 3, a porous sintered disc 4, a holding member 5, a peelable plastic film 6 and a cover 7.

The device also includes two check valves 8 and 9 and a female insert 10.

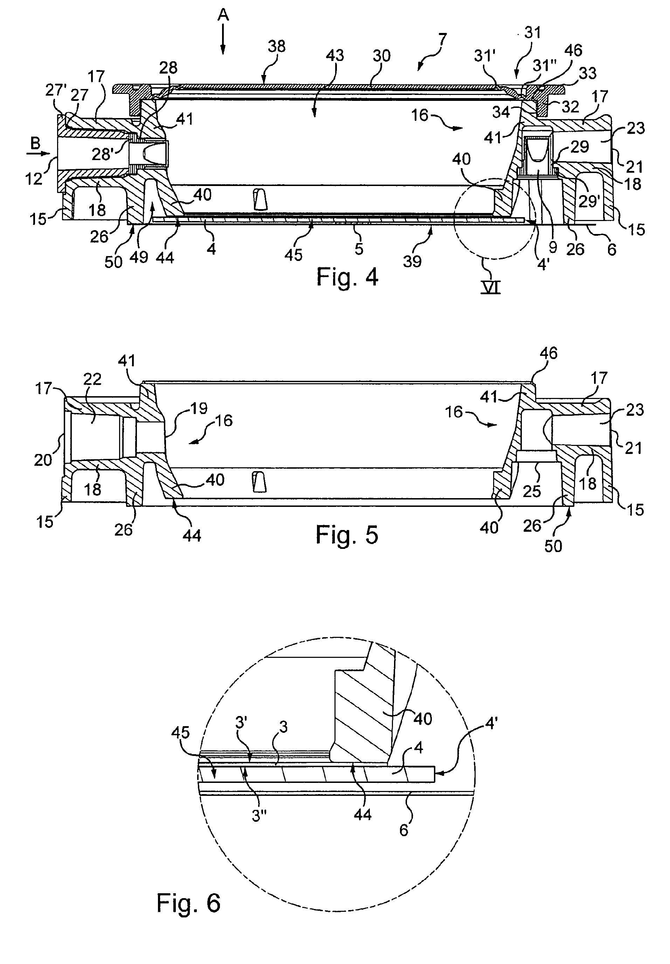

The body 2 is molded in one piece from polycarbonate. This body, shown separately in figure 5, has a globally cylindrical external wall 15 and a frustoconical internal wall 16 converging toward the membrane 3.

The wall 15 has two identical depressions 47 and 48 (figure 1) obtained by local deformation of the wall.

The walls 15 and 16 are joined together by walls 17 and 18. The wall 16 has an orifice 19 between the greatest diameter portion 41 of the wall 16 and its smallest diameter portion 40.

The orifice 19 communicates by a passage 22 between the walls 17 and 18 with an orifice 20 in the cylindrical wall 15.

A second orifice 21 in the wall 15 diametrically opposite the orifice 20 is extended by a passage 23 between the walls 17 and 18. The passage 23 includes an elbow portion such that, at the end opposite the orifice 21, it discharges into the space situated between the walls 16 and 15 via an orifice 25 in the wall 18.

A cylindrical intermediate wall 26 disposed between the walls 15 and 16 and projecting on the side opposite the passages 22 and 23 is joined transversely to the wall 18.

The valve 8 is nested inside the passage 22 at the level of the wall 16, being centered about its flange 28 with its flange 28' butted up against the body 2.

In the same way, the valve 9 is nested in the passage 23, being centered about its flange 29, with its flange 29' butted up against the wall portion 18 around the opening 25.

The valves 8 and 9 are set to open only beyond a certain pressure value when a fluid is supplied under pressure in the flow direction from the inlet orifice 20 to the outlet orifice 21 and to remain closed otherwise.

When the valve 8 has been inserted, the insert 10 is nested inside the passage portion 22 situated at the same end as the orifice 20, centered around its flange 27, with its annular flange 27' butted up against the external surface of the cylindrical wall 15 so that the orifice 12 of this insert, opposite the valve 8, is situated in the vicinity of the orifice 20 in the body 2.

Here the insert 10 is a Luer female connector, a seal being obtained between this connector and the body 2 by ultrasound welding around the perimeter of the insert.

Like the insert 10, the passage 23 and the walls around it also form a Luer female connector.

Each of the orifices 12 and 21 is closed by a peelable flexible plastic material tongue (not shown) to guarantee the integrity of the passages 22 and 23 by protecting them from the atmosphere external to the device, which is a potential source of microbiological contamination.

The flexible plastic material cover 7 is forcibly nested inside the portion 41 of the frustoconical wall 16.

The body 2 also includes a pointed annular rib 46 on the edge of the frustoconical portion 41 facing outwardly from the device and adapted to provide a seal with the cover (see below).

The cover 7 shown in figures 1 to 4 is of polypropylene and includes a closure wall 30, an annular hinge 31, a cylindrical nesting strip 32 and an annular flange 33.

The closure wall 30 is connected by the annular hinge 31 to the cylindrical nesting strip 32 and to the flange 33.

In its position nested against the frustoconical wall portion 41, the cylindrical strip 32 bears against the external surface of that portion through the intermediary of an annular rib 34.

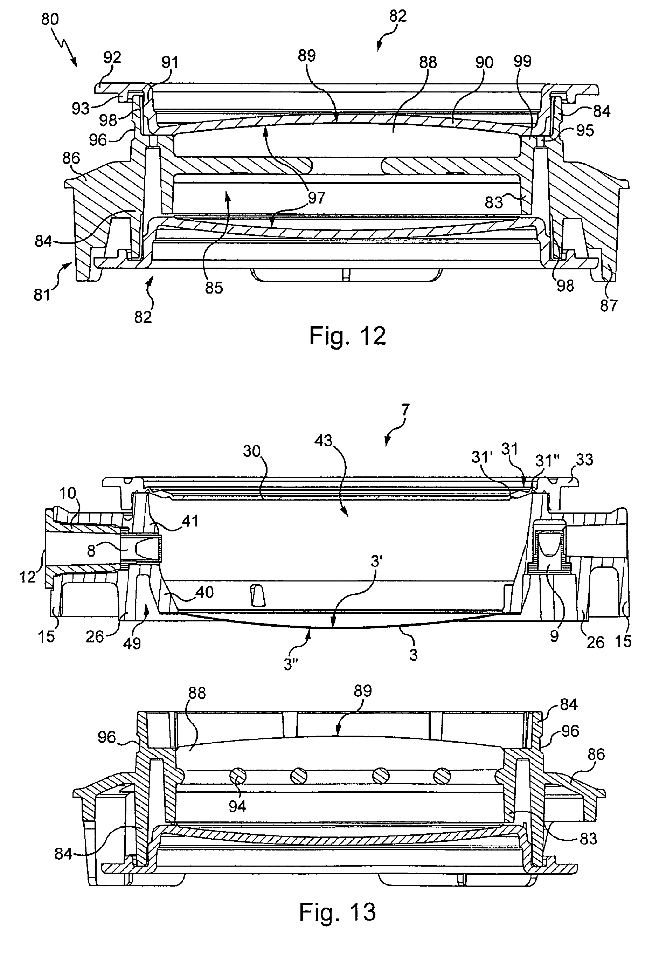

The annular hinge 31 connecting the wall 30 to the remainder of the cover has thinner portions 31' and 31" adapted to flex so that the cover 7 has a stable first position (figure 4) in which the wall 30 is at the level of the flange 33 and a stable second position (figures 13 to 15) in which that wall is substantially level with the rib 34. This is explained hereinafter.

The 55 mm diameter membrane 3 is of cellulose ester (it can equally be made of polycarbonate or PVDF). In particular, this material allows liquids to pass through but retains microorganisms that they contain.

The periphery of this membrane is sealed to the edge 44 of the frustoconical wall portion 40, this portion forming an annular rim.

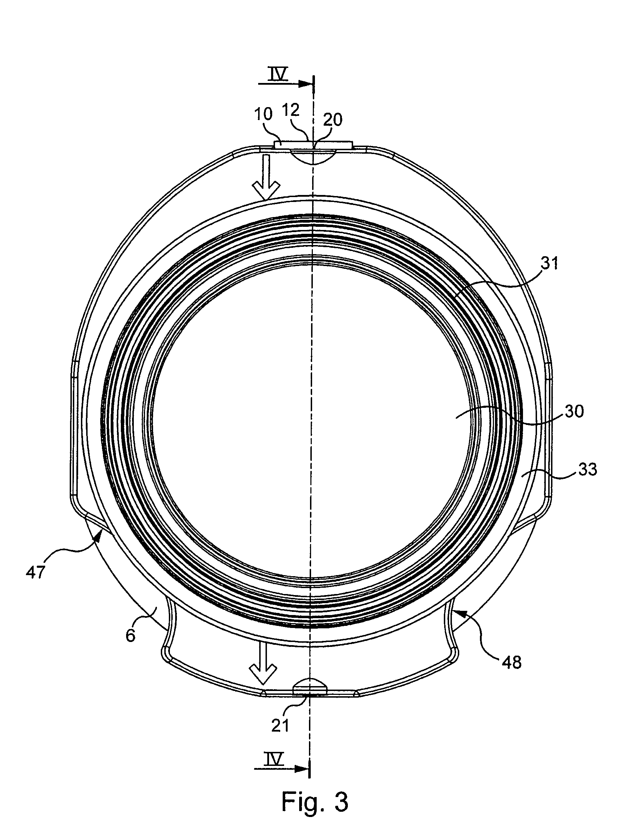

Under this membrane is disposed the porous sintered disk 4, which has a diameter larger than that of the membrane and thus is disposed against the rim 40, projects beyond this rim and supports the membrane over the whole of its surface 3" (figures 3 and 6) .

The holding member 5 centered relative to the edges of the sintered disk is disposed under the sintered disk.

Here the holding member 5 is a simple central stud that may be replaced with peripheral studs or even a disk, for example.

The porous sintered disk 4 is supported via the stud 5 by a polyethylene film 6 sealed to the annular edge 50 of the cylindrical wall 26, so that this film entirely covers and hermetically seals the stud 5, the porous sintered disk 4 and the membrane 3, with the stud 5 holding the sintered disk 4 in position against the membrane 3 and the annular rim 40.

Strips of this film (not visible in the figures) are sealed to the sintered disk 4 to fasten together the film, the sintered disk and the stud disposed between them.

The test device ready for use (with the cover 7 nested in the body 2 and the film 6 sealed to that body) therefore has two flexible and detachable transverse walls (the wall 30 associated with the hinge 31 and the film 6) that are globally parallel to each other, closing the top and the bottom of the filtration chamber delimited by the body 2, the wall 30 (respectively the film 6) having a face 38 (respectively 39) facing outwardly from the device.

The wall 30 and the film 6 are held by simple adhesion phenomena (by friction as a result of force-fitting in the case of the wall 30, thanks to the strip 32 and the rib 34, and by cementing or hermetic bonding in the case of the film 6).

As explained hereinafter, the cover 7 more precisely closes a receiving volume 43 for the liquid situated between the membrane 3, the frustoconical wall 16 and the cover, and the plastic film 6 partially delimits with the membrane 3 a drainage volume 45 for the liquid situated under the membrane and including the space situated between the membrane and the film 6. This volume communicates with the annular volume 49 situated between the wall portion 40 and the wall 26 forming a volume through which the liquid passes after it has been received in the receiving volume 43 and then drained into the drainage volume 45, so that the liquid is guided to the outlet orifice 21 by the passage 23.

This device, previously sterilized by means of gamma radiation, is packaged in a plastic sachet (not shown) consisting of two thermoplastic sheets joined together by a weld bead, a section of that weld bead being peelable by hand.

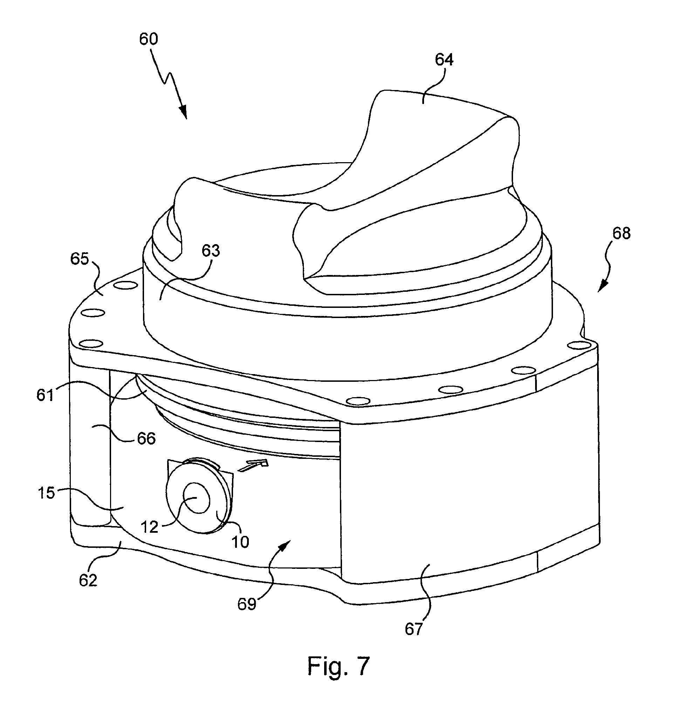

The clamp 60 is described next with reference to figures 7 to 9.

The clamp 60 includes a mobile plate 61, two fixed plates 62 and 65, a knob 64, a clamping mechanism 63 and two walls 66 and 67.

Each of the fixed plates 62 and 65 is mounted against a respective edge of the walls 66 and 67, which are spaced from each other with the result that the clamp 60 has a first window 68 between the walls 66 and 67 and the plates 62 and 65, at the rear in figure 7, and a second window 69 at the front in that figure.

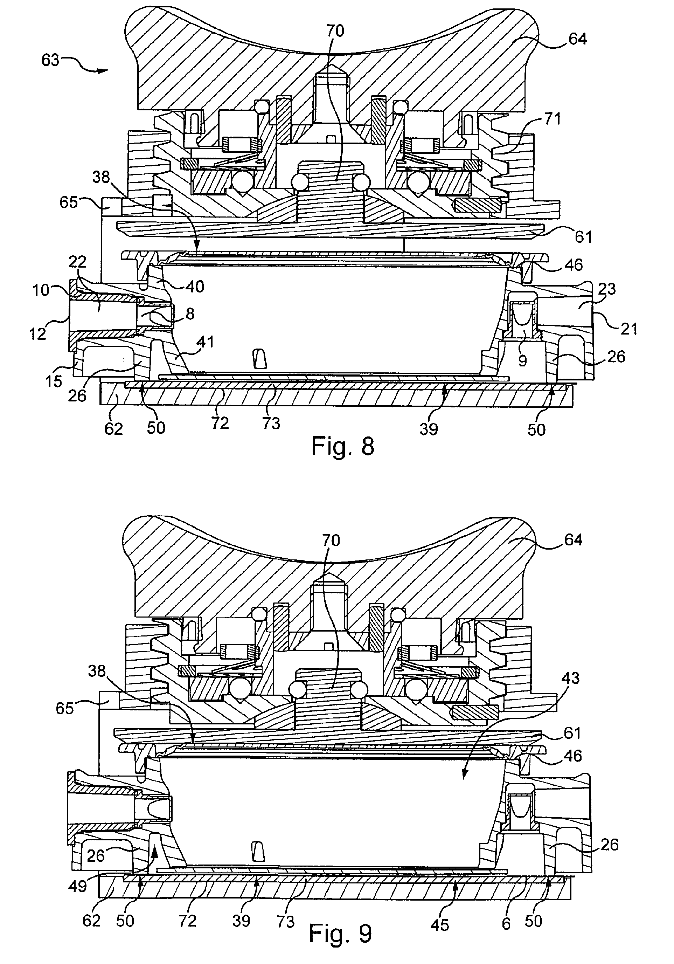

The mobile plate 61 is disposed between the plates 62 and 65 and is connected to the mechanism 63 by a rod 70 passing through an orifice in the plate 65 (figures 8 and 9) .

The fixed plate 62 has at its center, on the side facing the plate 65, a cylindrical cavity 72 in which is deposited an elastic and flexible material 73 such as silicone.

The clamping mechanism 63 is connected by a screw to the knob 64 and includes a toothed rack 71 adapted to move the mobile plate 61 in translation between the plates 62 and 65 when an operator turns the knob 64 and to hold this plate in position despite any pressure forces exerted on it, for example by the device 1 (see below).

The mechanism 63 is a torque limiter mechanism enabling the device 1 to be clamped with a predetermined force.

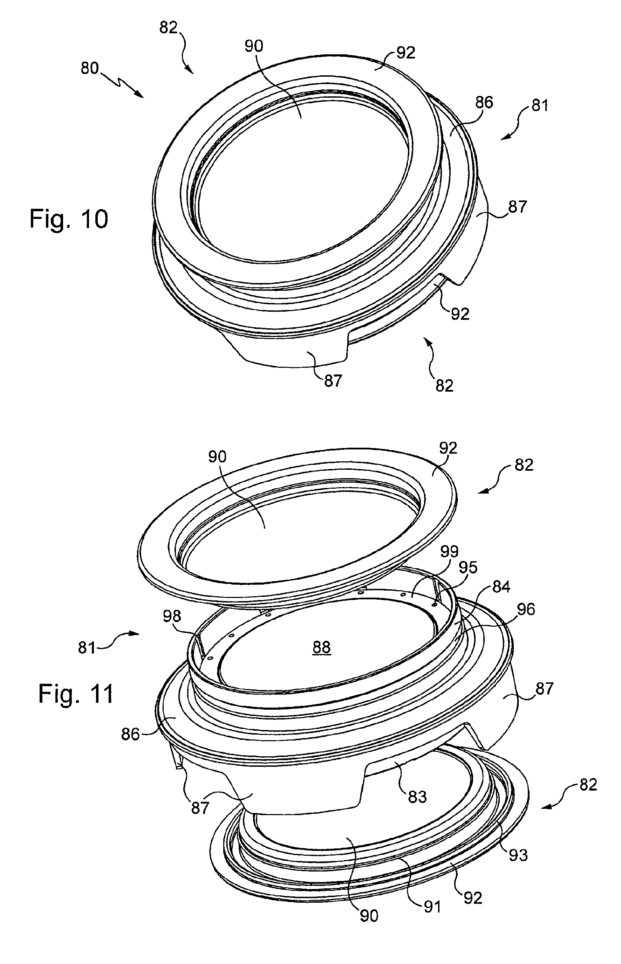

The gel growth medium cassette 80 is described next with reference to figures 10 to 12.

The gel growth medium cassette 80 has a cassette body 81 and two identical covers 82. The body 81 is of generally cylindrical shape and has a first cylindrical wall 83, a second cylindrical wall 84, a mesh support plate 85, a balcony 86 and a plurality of teeth 87.

The wall 84 surrounds the wall 83 and is connected to it by a transverse wall 99.

A series of rods 94 upstanding from the surface of the wall 83 facing toward the interior of the cassette in the direction of the geometrical center of that cylindrical wall are connected by circular ribs (not shown) to form the mesh support 85.

The balcony 86 is connected to the wall 84 on the side opposite the wall 99. It has a series of regularly spaced teeth 87 projecting parallel to the wall 84.

An annular groove 96 on the face of the wall 84 carrying the balcony 86, at the level of the transverse wall 99, is adapted to cooperate with the device 1 (see below) .

Each of the covers 82 has a domed wall 90 connected by a cylindrical strip 91 to a flange 92.

The flange 92 has an annular rib 93 projecting on the same side as the strip 91.

An Agar-based gel growth medium 88 is poured onto the mesh 85 of the cassette 60 (which at this time is upside down compared to figure 12 with the cover 82 situated in the vicinity of the teeth 87 removed) so as to coat the mesh and to have a convex surface 89 in the direction of the cover 82 that has not been removed.

Each cover 82 of a gel growth medium cassette ready for use (figure 12) is nested against a respective free edge of the wall 84 (these edges are then disposed between the strip 91 and the rib 93 of the corresponding cover 82) so that the concave surfaces 97 of the covers face toward the mesh support 85, the concave face 97 of domed wall 90 of the cover 82 closer to the gel growth medium extending along the convex surface 89 of the gel growth medium, thereby protecting it from the atmosphere.

To this end the wall 99 has orifices 95 forming a vent to prevent the gel growth medium 88 sticking to the cover 82 by suction when it is removed.

Similarly, the wall 84 has a plurality of grooves 98 to enable air to pass between the second cover 82 and the gel growth medium 88.

The gel growth medium cassette is designed so that, to simplify storage, it may be nested with other identical gel growth medium cassettes with the top cover of the lower cassette resting against the bottom cover of the higher cassette and the free edges of the teeth 87 of the upper cassette partially surrounding the two covers to prevent one cassette slipping relative to another.

How a microbiological test is carried out using a device of the invention is described next.

Initially, the operator opens the individual sachet in which the device 1 is contained (by pulling the two thermoplastic films apart at the peelable weld bead) to extract it by gripping the depressions 47 and 48 of the body 2.

The device 1 is then engaged in the clamp 60 via the window 68, orifice 12 first, so that it butts up against the wall portions 66 and 67 situated in the vicinity of the window 69, which is narrower than the window 68.

Thus the device 1 is disposed between the fixed plate 62 and the mobile plate 61 with the edge 50 of the wall 26 to which the film 6 is sealed bearing on the silicone 73 filling the cavity 72 in the plate 62 (figures 8 and 9).

The operator then turns the knob 64 to operate the clamping mechanism 63 and clamp the device 1, the mobile plate 61 coming to bear against the surface 38 of the cover 7 and the fixed plate 62 coming to bear against the surface 39 of the film 6.

When the device 1 is clamped sufficiently tightly, the torque limiter of the mechanism 63 disengages the knob 64 so that the operator cannot clamp the device 1 more tightly. The torque limiter is set so that the clamping pressure is sufficient to seal the device 1 without crushing it unduly.

The wall 26 deforms the silicone locally to guarantee a perfect seal between the wall 26 and the film 6 (figure 9) .

The seal with the body 2 on the side with the cover 7 is guaranteed by the annular rib 46, which the clamp 60 presses onto a flexible portion of the cover 7.

Once the device has been clamped, the operator peels off the plastic film (not shown) closing off the inlet orifice 12 of the device and then, through the window 69, connects the Luer female inlet connector 10 of the device to a male Luer connector (not shown) connected to a filling passage communicating via a valve (not shown) with a tank of liquid under pressure (not shown).

The operator then peels off the plastic film closing off the outlet orifice 21 to connect the outlet female Luer connector to a drainage passage (not shown) through the window 68.

The operator then maneuvers the valve so that the filter chamber is at the same pressure as the liquid, for example 3 bar. The clamp 60 guarantees that the device 1 is sealed for pressures as high as 8 bar.

The liquid then passes through the passage 22 in the direction of the arrow B (figure 4), its pressure being sufficient to open the valve 8, and then fills the receiving volume 43 and begins to pass through the entire thickness of the membrane 3 in the axial filtering direction represented by the arrow A (figure 4).

Because this membrane is hermetically sealed to the edge 44 of the rim 40, the liquid can escape from the receiving volume 43 only by passing through the entire thickness of the membrane 3.

Once the liquid has been filtered through the membrane, it enters the drainage volume 45 in the porous sintered disk 4 and passes at least partially through the latter.

The major portion of the liquid passes through only a portion of the thickness of the sintered disk, escaping from the disk via its edge 4' (figure 6).

The drainage volume is therefore essentially localized within the volume occupied by the sintered disk 4 and within the volume extending radially (i.e. transversely relative to the axial filtration direction) around the sintered disk situated in the vicinity of the edge 4'.

It is therefore clear that the sintered disk could instead be connected directly to the film 6 with no intermediate holding member such as the stud 5.

The liquid then moves from the drainage volume 45 toward the transit volume 49 situated between the rim 40 and the wall 26 radially distributed around the membrane 3, and the liquid is therefore conveyed to the valve 9.

Like the valve 8, this valve is adapted to open at the working pressure of the device, and the liquid can therefore be evacuated via the orifice 21, flowing through the passage 23.

Should the operator interchange the inlet and outlet orifices by mistake, the check valves 8 and 9 prevent the liquid from passing through the device in the direction that would cause a pressure difference across the membrane 3 that would deform it on the side opposite the sintered disk supporting it and possibly tear it.

Once all of the liquid has been filtered, the operator closes the liquid inlet valve, disconnects the filler and drain passages of the device 1 and extracts the device 1 from the clamp 60. With the device extracted from the clamp, the membrane 3 charged with water is supported only by the sintered disk 4, to prevent it tearing.

The operator then purges the liquid contained in the device by connecting the outlet orifice 21 of the device to a vacuum pump to apply a reduced pressure via the passage 23 to aspirate the liquid.

When this purging operation has been completed, the operator grasps the peelable film 6 near one of the depressions 47 and 48 and peels the film off (figure 1), the stud 5 and the porous sintered disk 4 being entrained by the film 6 and removed with it (the film being sealed to the sintered disk at several places), only the membrane 3 remaining fastened to the rim 40.

The operation of clipping the filter device 1 to the gel growth medium cassette 80 is described next with reference to figures 13 to 16.

Initially, the operator removes the cover 82 protecting the gel growth medium 88 by grasping the flange 92 of the cover to disengage it from the wall 84 (figure 13).

Next, he applies pressure to the surface of the cover 7 of the device 1 in the vicinity of its center in order to flex the thinner portions 31' and 31" of the hinge 31, the wall 30 of this cover and the hinge 31 then going from their first stable position represented in figure 13 to their second stable position, reducing the receiving volume 43, at which time the operator can release the pressure applied to the cover.

The valve 8 and the membrane 3 forming an airtight device, the reduction of the receiving volume 43 causes a slight pressure rise in the device 1, this pressure rise being sufficiently small for the cover to remain in a second stable position even if the operator is no longer pressing on it.

In response to this pressure rise, the membrane 3, which is retained at its periphery, is deformed to a slightly domed shape (its surface 3' becomes concave and its surface 3" becomes convex) towards the exterior of the device (figure 13).

The operator then places the device on top of the cassette 80 so that the convex surface 3" of the membrane faces the convex surface 89 of the gel growth medium, continuing to press on the cover 7.

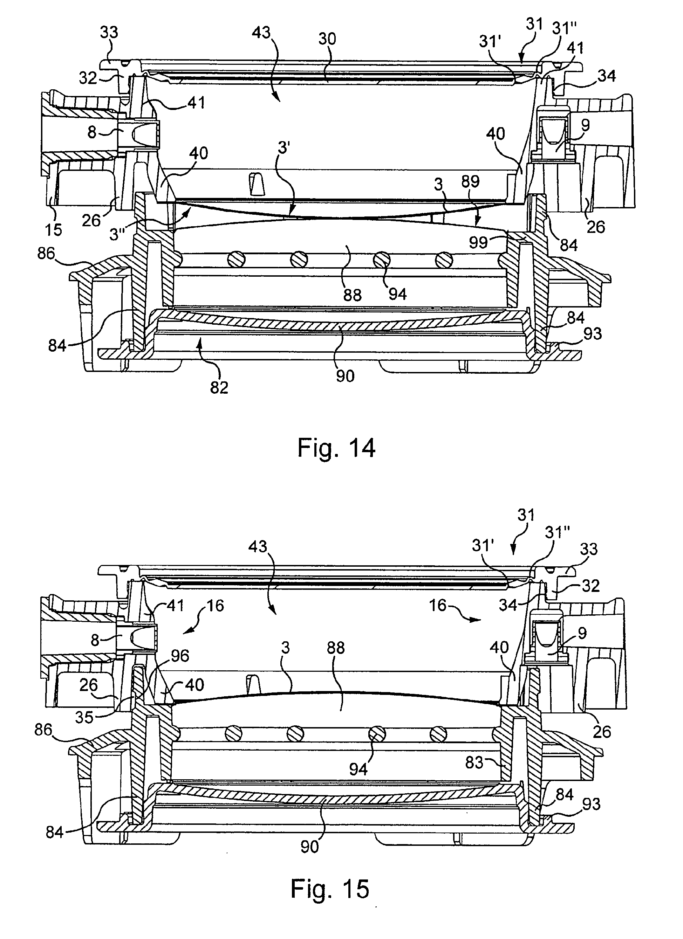

The operator then brings the membrane 3 and the gel growth medium 88 into contact, the center of the surface 3" contacting the center of the concave surface 89 of the gel growth medium (figure 14). The wall 84 of the cassette 80 is engaged between the walls 26 and 40 of the device to center and guide the device 1 relative to the cassette.

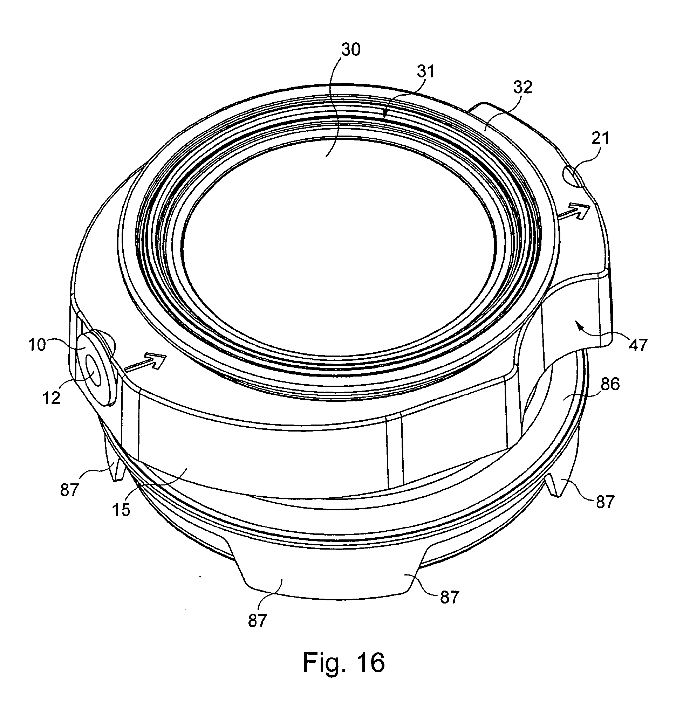

The operator continues this movement until the device 1 is clipped to the cassette 60 (figures 15 and 16).

During this movement, the membrane is pressed progressively onto the gel growth medium, expelling toward the periphery of the membrane residual air present between the membrane and the gel growth medium.

The progressive application of the membrane to the gel growth medium, first at its center and from there out to its periphery, avoids the formation of residual air pockets between the membrane and the gel growth medium that could locally impede the growth of microorganisms.

At the end of this step, regularly spaced bosses 35 of the device 1 projecting from the wall 26 toward the wall 16 of the device 1 (figure 15) clip into the groove 96 on the cassette 80, which fastens together the assembly formed by the device 1 and the cassette 80 and holds it in a stable position.

This assembly is then placed to incubate in an incubation chamber for the time necessary to grow the microorganisms retained in the membrane 3 so that they are visible and can be counted.

Several assemblies can be stacked up in the incubation chamber to facilitate storage.

The assemblies may also be turned over to limit condensation on the gel growth medium.

Once incubation has been carried out, the colonies are counted through the cover if it is transparent or the cover 7 is removed and the colonies counted directly.

In a variant, the cavity 72 in the fixed plate 62 remains empty to enable the film 6 to deform locally into this cavity to facilitate the flow of liquid from the drainage region to the transit region, the drainage volume in this case being also formed of the volume situated between the porous sintered disk 4 and the film 6 deformed by the pressure of the liquid.

In another variant, the cover 7 of the device is replaced by a flexible wall that is not detachable (and is adapted to become domed in the direction of the membrane) or by a peelable film similar to the film 6.

In a further variant, the film 6 is replaced by a cover similar to the cover 7 or by a tearable film.

In a further variant, the porous sintered disk is replaced by an impermeable support plate in which a plurality of passages is formed extending radially from the center of the plate toward its periphery to enable drainage of the liquid under pressure.

The present invention is not limited to the embodiment described and shown and encompasses any variant execution thereof.

## Claims

1. Device for microbiological testing of a sample of liquid flowing under pressure in an axial filtration direction (A), the device including:
- a first body (2) having an inlet orifice (20) and a receiving volume (43) connected to said inlet orifice (20);
- an outlet orifice (21) and a drainage volume (45) communicating with said outlet orifice (21);
- a filter membrane (3) disposed transversely to said axial direction (A) between the receiving volume (43) and the drainage volume (45);
- a support member (4) removable from said first body (2) and adapted to restrict deformation of the membrane (3) when the liquid flows from the receiving volume (43) to the drainage volume (45) without blocking that flow; and
- a second body (6) removably fixed to said first body (2) to delimit the drainage volume (45) conjointly with the membrane (3);
**characterized in that**:
- the outlet orifice (21) is at the periphery of said first body (2); and
- said second body (6) is primarily formed of a detachable wall mounted by adhesion to said first body (2).

2. Device according to claim 1, **characterized in that** at least the major portion of said drainage volume (45) is formed in said first body (2).

3. Device according to either claim 1 or claim 2, **characterized in that** said detachable wall (6) holds said support member (4) in position inside said first body (2).

4. Device according to claim 3, **characterized in that** said detachable wall (6) is sealed to said support member (4).

5. Device according to any one of claims 1 to 4, **characterized in that** said wall of the second body (6) is flexible.

6. Device according to claim 5, **characterized in that** said wall is a peelable film (6).

7. Device according to any one of claims 1 to 5, **characterized in that** said detachable wall is part of a cover (7) force-fitted into or onto said first body (2).

8. Device according to any one of claims 1 to 7, **characterized in that** said first body (2) has on the opposite side of the wall of the second body (6) to said membrane (3) a second detachable wall (30, 31) mounted by adhesion to said first body (2) and closing said receiving volume (43).

9. Device according to claim 8, **characterized in that** said receiving volume forms a closed space in the presence of said second detachable wall (30, 31).

10. Device according to either claim 8 or claim 9, **characterized in that** said second wall (30, 31) is flexible.

11. Device according to claim 10, **characterized in that** said second wall is a peelable film.

12. Device according to any one of claims 8 to 10, **characterized in that** said second wall (30, 31) is part of a cover (7) force-fitted into or onto said first body (2).

13. Device according to any one of claims 8 to 12, **characterized in that** said second wall includes a flexible hinge (31).

14. Device according to claim 13, **characterized in that** said hinge (31) includes at least one portion of thinner material (31', 31").

15. Device according to claim 12, **characterized in that** said cover (7) has a first stable position in which said receiving volume (43) occupies a predetermined space and a second stable position in which said receiving volume (43) occupies a smaller space than said predetermined space.

16. Device according to any one of claims 8 to 15, **characterized in that** said walls (6, 30) mounted by adhesion to said first body (2) are substantially parallel to each other.

17. Device according to any one of claims 1 to 7, **characterized in that** said first body (2) has on the opposite side of the wall of the second body (6) to said membrane (3) a second wall (30) parallel to said detachable wall (6).

18. Device according to any one of claims 1 to 17, **characterized in that** the periphery of said membrane (3) is held against an annular rim (40) of said first body (2) between said receiving volume (43) and a peripheral transit volume (49) through which said drainage volume (45) communicates with the outlet orifice (21).

19. Device according to claim 18, **characterized in that** said membrane (3) is sealed to said annular rim (40).

20. Device according to either claim 18 or claim 19, **characterized in that** said rim (40) is part of a globally frustoconical wall (16) that converges axially toward the membrane.

21. Device according to any one of claims 18 to 20, **characterized in that** the transit volume (49) is situated between said rim (40), a lateral wall (26) around said rim (40) and a transverse wall (18) connecting said rim (40) to said lateral wall (26) on the opposite axial side to the detachable wall (6).

22. Device according to any one of claims 18 to 21, **characterized in that** said drainage volume (45) extends transversely beyond the annular rim (40) to join said peripheral transit volume (49).

23. Device according to claim 22, **characterized in that** said support member (4) is a porous member that extends laterally at least to a position facing said rim (40) and which extends axially over more than half of the distance between said membrane (3) and said detachable wall (6).

24. Device according to any one of claims 1 to 23, **characterized in that** said first body (2) has an annular shape that is globally symmetrical in the axial direction (A).

25. Device according to any one of claims 1 to 22, **characterized in that** said support member (4) is porous.

26. Device according to any one of claims 1 to 25, **characterized in that** it includes a holding member (5) disposed between said support member (4) and said detachable wall (6).

27. Device according to any one of claims 1 to 26, **characterized in that** said device includes a first calibrated valve (8) disposed between said inlet orifice (20) and said receiving volume (43) and a second calibrated valve (9) disposed between said drainage volume (45) and said outlet orifice (21), said valves (8, 9) being adapted to open at said flow pressure of the liquid in the flow direction from said inlet orifice (20) to said outlet orifice (21).

28. Assembly for microbiological testing of a sample of liquid flowing under pressure including a device according to any one of claims 1 to 27 and a clamp (60) having clamping means (61, 62, 63, 64) adapted to hold said device axially in the manner of a vice by said first body (2) and said detachable wall (6).

29. Assembly according to claim 28, **characterized in that** said clamp (60) exerts a clamping pressure on said detachable wall (6).

30. Assembly according to claim 29, **characterized in that** said clamp (60) has a fixed plate (62) and a plate (61) mobile between a rest position in which it is moved away from the device and a clamping position in which it bears on said device.

31. Assembly according to claim 30, **characterized in that** said clamping means (61, 62, 63, 64) include a knob (64) and a clamping mechanism (63), said knob (64) being adapted to be operated to operate said clamping mechanism (63) to move the mobile plate (61) from its rest position to its clamping position and vice versa.

32. Assembly according to claim 31, **characterized in that** said clamping mechanism (63) is adapted to limit the clamping torque when said knob (64) is operated so that said clamp (60) exerts a predetermined clamping pressure on said device.

33. Assembly according to any one of claims 30 to 32, **characterized in that** the plate (62) that engages said detachable wall (6) has a central cavity (72).

34. Assembly according to claim 33, **characterized in that** said cavity (72) is filled with an elastic and flexible material.

35. Assembly according to claim 34, **characterized in that** said material is a silicone (73).

36. Microbiological incubation assembly including a device according to any one of claims 1 to 27 and a gel growth medium cassette (80) including a body (81) surrounding said gel growth medium (88) and a support (85) on which said gel growth medium (88) rests, having on the side opposite said support a convex surface (89), the device being adapted to be nested with said cassette (80) after removal of said detachable wall (6) and of said support member (4) so that, in the position of nesting of said device in said cassette (80), the surface (3") of the membrane (3) of said device facing said cassette (81) rests on said gel growth medium (88).

37. Assembly according to claim 36, **characterized in that** said support (85) is a mesh onto which said gel growth medium (88) is poured.

38. Assembly according to either claim 36 or claim 37, **characterized in that** said device and said cassette (80) include complementary clipping means.

39. Assembly according to claim 38, **characterized in that** said complementary clipping means include at least one boss (35) forming part of said device and one groove (96) forming part of said cassette (80).

40. Method for microbiological testing of a sample of liquid flowing under pressure, including steps of:
- procuring a test device according to any one of claims 1 to 27;
- mounting said device in a clamp (60) to constitute a test assembly according to any one of claims 28 to 35;
- connecting the inlet orifice (20) of said device to a filler passage and the outlet orifice (21) to a drainage passage for said liquid;
- filtering a volume of liquid through said device;
- removing said device from said clamp (60);
- purging the liquid from said device;
- removing said detachable wall (6) and said support member (4) from said device;
- nesting said device on a gel growth medium cassette (80) to constitute an incubation assembly according to any one of claims 36 to 39; and
- waiting for incubation to take place.

41. Assembly according to claim 40, **characterized in that** the step of procuring a test assembly includes the step of selecting as the test device a device having a first body (2) including at least one flexible wall (30, 31) on the opposite side of the detachable wall (6) of the second body to said membrane (3) and **in that** said method includes, prior to the step of nesting the device to the gel growth medium cassette (80), the steps of:
- pressing on said first body (2) to flex said wall (30, 31) toward the interior of said device so that said membrane (3) is domed toward the exterior of said device; and
- pressing the membrane (3) onto the gel growth medium (88), bringing it into contact with the gel growth medium (88) firstly at its center and then progressively over the whole of its surface out to its periphery.

42. Method according to either claim 40 or claim 41, **characterized in that** the step of purging said liquid includes the step of connecting the outlet orifice (21) of said device to a vacuum supply and the step of aspirating said liquid through said outlet orifice (21).
